# FASCICULE DE BREVET EUROPEEN

(11) **EP 2 119 431 B1**
(45) Date de publication et mention de la délivrance du brevet: **15.11.2017**
(21) Numéro de dépôt: 09154178.9
(22) Date de dépôt: 03.03.2009
(51) Int. Cl.: A61K 8/44, A61K 8/63, A61K 8/97, A61Q 7/00, A61P 17/14

(54) **Composition contenant l'association de madécassoside et/ou de terminoloside et d'une arginine et/ou l'un de ses sels et/ou l'un de ses dérivés; utilisation de l'association pour induire et/ou stimuler la croissance de ces fibres kératiniques humaines et/ou freiner leur chute.**
Zubereitung enthaltend die Wirkstoffkombination aus Madecassoside und/oder Terminoside und Arginin und/oder dessen Salz und/oder dessen Derivaten; Verwendung dieser Wirkstoffkombination zur Stimulation oder Induktion des Wuchses keratinischer Fasern und/oder zur Bremsung deren Ausfalls.
Composition containing the combination of madecassoside and/or of terminoloside and of an arginine and/or a salt thereof and/or a derivative thereof; use of the combination for inducing and/or stimulating the growth of human keratin fibres and/or preventing their loss

(30) Priorité: 28.03.2008 FR 0852009
(43) Date de publication de la demande: 18.11.2009
(73) Titulaire: L'Oréal, 75008 Paris (FR)
(72) Inventeur: Lazzeri-Vigouroux, Pascale, 13540, PUYRICARD (FR); Rougier, André, 95550, BESSANCOURT (FR)
(74) Mandataire: Miszputen, Laurent

(56) Documents cités:
- EP-A1- 1 140 000
- WO-A-99/13717
- DE-A1-102004 041 588
- FR-A- 2 609 390
- US-A1- 2007 154 425
- AZIZ Z A ET AL: "Production of asiaticoside and madecassoside in Centella asiatica in vitro and in vivo" 1 mars 2007 (2007-03-01), BIOLOGIA PLANTARUM, KLUWER ACADEMIC PUBLISHERS, DO, PAGE(S) 34 - 42 , XP019524214 ISSN: 1573-8264 * le document en entier *
- BONTE F ET AL: "ACTIVITE COMPAREE DE L'ASIACOSIDE ET DU MADECASSOSIDE SUR LA SYNTHESE DES COLLAGENES I ET III PAR DES FIBROBLASTES HUMAINS EN CULTURE//ASIATICOSIDE AND MADECASSOSIDE ACTIVITY ON HUMAN FIBROBLAST TYPE I AND III COLLAGEN SECRETION" 1 janvier 1995 (1995-01-01), ANNALES PHARMACEUTIQUES FRANCAISES, MASSON, PARIS, FR, PAGE(S) 38 - 42 , XP008064824 ISSN: 0003-4509 * le document en entier *
- LEPETIT J-C: "MADECASSOSIDE - IMMUNE REGULATION OF PSORIASIS-LIKE DISORDERS BY NATURAL ANTI-INFLAMMATORY ACTIVE INGREDIENT" 1 avril 2005 (2005-04-01), SOFW-JOURNAL SEIFEN, OELE, FETTE, WACHSE, VERLAG FUR CHEMISCHE INDUSTRIE, AUGSBURG, DE, PAGE(S) 28,30,32,34 , XP008060903 ISSN: 0942-7694 * le document en entier *

## Description

### DOMAINE DE L'INVENTION

L'invention a pour objet un procédé de soin des fibres kératiniques humaines et notamment destinée à diminuer ou à freiner leur chute et/ou à induire et/ou à stimuler leur croissance et/ou augmenter leur densité, contenant dans un milieu physiologiquement acceptable au moins
a) une arginine et/ou bien l'un de ses sels d'acide organique ou minéral et/ou l'un de ses dérivés ;
b) un composé ou un mélange de composé de formule (I) ou bien un extrait de Centella Asiatica les comprenant.
Elle se rapporte, en outre, à un procédé de traitement cosmétique destiné à induire et/ou stimuler la croissance des fibres kératiniques humaines et/ou freiner leur chute et/ou augmenter leur densité ainsi qu'à l'utilisation de ladite association pour induire et/ou stimuler la croissance de ces fibres kératiniques et/ou freiner leur chute.
Les fibres kératiniques humaines auxquelles s'applique l'invention sont notamment les cheveux, les sourcils, les cils, les poils de barbe, de moustache et les poils pubiens. Plus spécialement, l'invention s'applique aux cheveux et/ou aux cils humains.
Chez l'être humain, la croissance des cheveux et leur renouvellement sont principalement déterminés par l'activité des follicules pileux et de leur environnement dermo-épidermique. Leur activité est cyclique et comporte essentiellement trois phases, à savoir la phase anagène, la phase catagène et la phase télogène.
A la phase anagène (phase active ou phase de croissance), qui dure plusieurs années et au cours de laquelle les cheveux s'allongent, succède une phase catagène très courte et transitoire qui dure quelques semaines. Au cours de cette phase, le cheveu subit une évolution, le follicule s'atrophie et son implantation dermique apparaît de plus en plus haute.
La phase terminale, qui dure quelques mois, correspond à une phase de repos, appelée phase télogène. A la fin de cette période de repos, les cheveux tombent et un autre cycle recommence.
La chevelure se renouvelle donc en permanence, et sur les 150 000 cheveux environ que comporte une chevelure, à chaque instant, 10% d'entre eux environ sont au repos et seront donc remplacés en quelques mois.
La littérature évoque de nombreuses raisons entraînant la chute précoce des cheveux ; en particulier cette chute précoce survient chez des sujets prédisposés génétiquement et elle atteint notamment les hommes. Il s'agit plus particulièrement de l'alopécie androgénétique ou androgénique ou encore androgéno-génétique.

Cette alopécie est essentiellement due à une perturbation du renouvellement capillaire qui entraîne, dans un premier temps, l'accélération de la fréquence des cycles aux dépens de la qualité des cheveux puis de leur quantité. Il se produit un appauvrissement progressif de la chevelure par régression des cheveux dits « terminaux » au stade de duvets. Des zones sont touchées préférentiellement ; notamment chez l'homme, les golfes temporaux ou frontaux ainsi que la partie supérieure de l'occipital, tandis que chez les femmes on constate plutôt une alopécie diffuse du vertex.

D'autres causes peuvent entraîner une importante, temporaire ou définitive, des cheveux. Il peut s'agir de chute et d'altération des cheveux après une grossesse (post partum), au cours d'états de dénutrition ou de déséquilibres alimentaires ou encore au cours d'états d'asthénie ou de dysfonctionnement hormonal comme cela peut être le cas au cours ou après la ménopause. Il peut également s'agir de chute ou d'altérations des cheveux en relation avec des phénomènes saisonniers.

### ARRIERE PLAN DE L'INVENTION

On recherche depuis de nombreuses années, notamment dans l'industrie cosmétique, des compositions permettant de supprimer ou de réduire l'effet de l'alopécie et notamment d'induire ou de stimuler la croissance des cheveux et/ou de certains poils et/ou diminuer ou retarder leur chute.

On connaît dans la demande WO99/13717, la D-arginine, la D,L-arginine, la L-arginine ainsi que leurs sels ou leurs dérivés comme actifs destinés au traitement cosmétique par application topique de la chute des cheveux. Leur efficacité reste cependant limitée.

Dans les demandes EP277455, FR2606634, JP-7010722, on sait que les extraits de Centella asiatica sont connus comme actifs pour la prévention de la chute des cheveux et/ou la pousse des cheveux. Dans la demande WO2005/123032, on a déjà proposé d'utiliser une association d'un extrait de Centella Asiatica, d'extrait de café et d'antioxydant dans des compositions destinées à la prévention de la chute des cheveux et/ou la repousse des cheveux. Leur efficacité reste cependant. limitée

Par ailleurs, la demande WO2004/062678 décrit un extrait de Centella Asiatica riche en madécassoside et en terminoloside et son utilisation dans la régulation des mécanismes inflammatoires.

La demanderesse a découvert de manière surprenante, au cours de ses recherches, que l'association a) d'une arginine et/ou de l'un de ses sels d'acide organique ou minéral et/ou l'un de ses dérivés et b) d'un composé ou d'un mélange de composé de formule (I) que l'on définira plus loin ou bien d'un extrait de Centella Asiatica comprenant le ou lesdits composés de formule (I) conduisait à une efficacité antichute substantiellement plus importante par rapport à chacun des actifs utilisés seuls

### EXPOSE DE L'INVENTION

L'invention a pour objet un procédé de soin des fibres kératiniques humaines comprenant dans un milieu physiologiquement acceptable :
a) un ou plusieurs composés choisis parmi les arginines et/ou leurs sels d'acide organique ou minéral et/ou leurs dérivés ;
b) un composé ou un mélange de composés de formule (I) ou bien un extrait de Centella Asiatica comprenant un composé ou un mélange de composés de formule (I).
L'invention se rapporte encore à l'utilisation cosmétique de l'association a) d'un ou plusieurs composés choisis parmi les arginines et/ou leurs sels d'acide organique ou minéral et/ou leurs dérivés et b) d'un composé ou un mélange de composés de formule (I) ou bien d'un extrait de Centella Asiatica comprenant un composé ou un mélange de composés de formule (I), dans une composition de soin des fibres kétatiniques humaines, dans le but d'induire et/ou de stimuler la croissance desdites fibres et/ou freiner leur chute et/ou augmenter leur densité.
Par augmenter la densité des fibres kératiniques humaines, et notamment la densité capillaire, on entend augmenter le nombre de fibres kératiniques, notamment de cheveux, par cm² de peau telle que le cuir chevelu. Ainsi, cette composition permet de maintenir en bon état la chevelure et/ou lutter contre la chute naturelle des cheveux en particulier des hommes.
En particulier, l'invention se rapporte à l'utilisation cosmétique de ladite association dans une composition de soin capillaire pour traiter l'alopécie d'origine naturelle et en particulier l'alopécie androgénique ou andro-chrono-génétique
La présente invention a également pour objet un procédé de traitement cosmétique des fibres kératiniques humaines et/ou du cuir chevelu, destiné à induire et/ou stimuler la croissance desdites fibres, en particulier les cheveux et certains poils comme les cils et/ou freiner leur chute et/ou augmenter leur densité, caractérisé par le fait qu'il consiste à appliquer sur ces fibres et/ou le cuir chevelu, une composition cosmétique telle que définie ci-dessus, à laisser celle-ci au contact de ces fibres kératiniques et/ou du cuir chevelu, et éventuellement à rincer ces fibres kératiniques et/ou le cuir chevelu.
Ce procédé de traitement présente bien les caractéristiques d'un procédé cosmétique dans la mesure où il permet d'améliorer l'esthétique des fibres kératiniques humaines et en particulier des cheveux et des cils en leur donnant une plus grande vigueur et un aspect amélioré. En outre, il peut être utilisé quotidiennement pendant plusieurs mois.
Les compositions conformes au procédé de l'invention comprennent comme premier actif une arginine et/ou bien l'un de ses sels et/ou bien l'un de ses dérivés.
Les différentes formes de l'arginine sont en général la D-arginine, la D,L-arginine et la L-arginine.

Parmi les sels d'acide organique ou minéral d'arginine, on peut citer par exemple les chlorhydrates, les glutamates, butyrates, et glycolates.
Par dérivés d'arginine on entend au sens de l'invention des arginines portant des substitutions soit sur le groupement carboxyle des dites arginines soit sur le groupement amino porté par le carbone en alpha du groupement carboxyle ou éventuellement sur ces deux groupements.
Parmi les dérivés de la L-arginine, on peut citer les C₁-C₄ alkyl esters de L-arginine, l'alkyle étant choisi par exemple parmi méthyle, éthyle, propyle, isopropyle, butyle, isobutyle.
Parmi les dérivés de la L-arginine, on peut également citer les oligomères de L-arginine (appelés polyarginine), tels que ceux décrits dans la demande EP1060739 et par dans la demande WO 03/072039,
Des oligomères d'arginine préférés selon l'invention comprennent de 7 à 15 motifs L-arginine, par exemple 7, 9, 11, 13 ou 15 motifs L-arginine.
On utilisera plus particulièrement la L-arginine.

La ou les arginines et/ou leurs sels et/ou leurs dérivés conformes au procédé de l'invention seront présents dans les compositions dans des quantités allant de préférence de 0,01% à 5% du poids total de la composition et plus préférentiellement en une quantité représentant de 0,1% à 5% du poids total de la composition, et encore plus préférentiellement de 1 % à5%.

Le ou les composés de formule (I) conformes au procédé de l'invention répondent à la formule suivante : 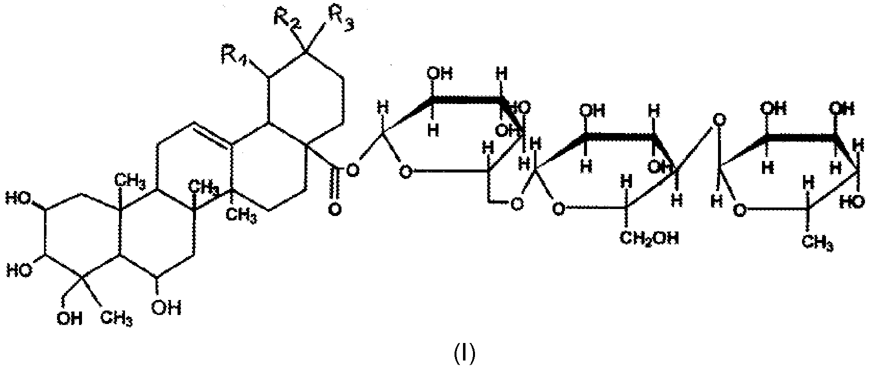 dans laquelle R₁ = H ou -CH₃; R₂ = H ou -CH₃; R₃ = -CH₃, R₁ et R₂ n'étant pas simultanément H.

Les composés de formule (I) préférés sont le madécassoside (composé de formule (I) avec R₁ = R₃ = -CH₃ et R₂ = H) et le terminoloside (composé de formule () avec R₁ = H et R₂ = R₃ = -CH₃).
Ces composés de formule (I) et notamment leurs mélanges, peuvent en particulier être extraits de Centella asiatica selon le procédé décrit dans la demande WO 2004/062678.
Selon un mode préféré de l'invention, on utilise un mélange de madécassoside et de terminoloside. Plus préférentiellement, le mélange de madécassoside et de terminoloside a une teneur en madécassoside allant de 30 à 70% en poids par rapport au poids total du mélange et plus particulièrement 50% en poids en madécassoside.
Selon un mode particulièrement préféré de l'invention, on utilisera un extrait de Centella asiatica comprenant plus de 95% en poids du mélange madécassoside / terminoloside par rapport au poids de l'extrait.
Un extrait de Centella Asiatica comprenant plus de 95% en poids de mélange de madécassoside / terminoloside (50/50% en poids) est notamment vendu sous la dénomination commerciale MADECASSOSIDE par la société BAYER. Ce mélange a pour nom CTFA MADECASSOSIDE.
Le composé ou le mélange de composés de formule (I) décrit précédemment peut être présent dans la composition pour application topique, en une quantité allant par exemple de 0,001 à 20 % en poids et de préférence de 0,01 à 5 % en poids par rapport au poids total de la composition.

### Milieu physiologiquement acceptable

Les compositions utilisées dans le procédé de l'invention peuvent constituer notamment des compositions cosmétiques et dermatologiques. Elles contiennent pour une telle application, un milieu physiologiquement acceptable. On entend ici par « milieu physiologiquement acceptable », un milieu compatible avec la peau, et éventuellement avec les lèvres, le cuir chevelu, les cils, les yeux et/ou les cheveux.

La composition utilisée dans le procédé l'invention peut appliquée par voie topique sur le cuir chevelu ou les fibres kératiniques humaines (sur toute zone cutanée ou fibres à traiter).
Cette composition peut se présenter sous toutes formes galéniques connues adaptées au mode d'utilisation.
Pour une application topique sur la peau, y compris le cuir chevelu, la composition peut avoir la forme d'une solution ou suspension aqueuse, alcoolique, hydro-alcoolique ou huileuse, d'une émulsion ou dispersion de consistance plus ou moins fluide et notamment liquide ou semi-liquide, obtenue par dispersion d'une phase grasse dans une phase aqueuse (H/E) ou inversement (E/H), d'une dispersion ou émulsion solide (H/E) ou (E/H), d'un gel aqueux, hydro-alcoolique ou huileux plus ou moins fluide ou solide, d'une poudre libre ou compactée à utiliser telle quelle ou à incorporer dans un milieu physiologiquement acceptable, ou encore de microcapsules ou microparticules, de dispersions vésiculaires de type ionique et/ou non ionique.
On peut également envisager une composition sous forme de mousse ou encore sous forme de spray ou d'aérosol comprenant alors un agent propulseur sous pression.
La composition peut ainsi se présenter sous forme d'une lotion, sérum, lait, crème H/E ou E/H, gel, onguent, pommade, poudre, baume, patch, tampon imbibé, savon, pain, mousse.
En particulier la composition à application sur le cuir chevelu ou les cheveux peut se présenter sous forme d'une lotion de soin capillaire, par exemple d'application journalière ou bi-hebdomadaire, d'un shampooing ou d'un après-shampooing capillaire, en particulier d'application bi-hebdomadaire ou hebdomadaire, d'un savon liquide ou solide de nettoyage du cuir chevelu d'application journalière, d'un produit de mise en forme de la coiffure (laque, produit pour mise en pli, gel coiffant), d'un masque traitant, d'une crème ou d'un gel moussant de nettoyage des cheveux. Elle peut encore se présenter sous forme de teinture ou de mascara capillaire à appliquer au pinceau ou au peigne.
Par ailleurs, pour une application sur les cils ou les poils, la composition à laquelle s'applique l'invention peut se présenter sous forme d'un mascara, pigmenté ou non, à appliquer à la brosse sur les cils ou encore sur les poils de barbe ou de moustache.

Selon un mode de réalisation particulier, la composition du procédé de l'invention se présente sous forme de crème ou lotion capillaire, de shampooing ou d'après-shampooing capillaire, de mascara capillaire ou pour cils.
Les quantités des différents constituants du milieu physiologique de la composition selon l'invention sont ceux généralement utilisées dans les domaines considérés. En outre, ces compositions sont préparées selon les méthodes usuelles.
Lorsque la composition est une émulsion, la proportion de la phase grasse peut aller de 1 % à 80 % en poids, et de préférence de 1 % à 50 % en poids par rapport au poids total de la composition. La phase aqueuse est ajustée en fonction de la teneur en phase grasse et en composé(s) (A) ainsi que de celle des éventuels ingrédients additionnels, pour obtenir 100% en poids. En pratique la phase aqueuse représente de 5 % à 99,9% en poids.
La phase grasse peut contenir des composés gras ou huileux, liquides à température ambiante (25°C) et pression atmosphérique (760 mm de Hg), généralement appelés huiles. Ces huiles peuvent être compatibles ou non entre elles et former une phase grasse liquide macroscopiquement homogène ou un système bi- ou triphasique.
La phase grasse peut, en plus des huiles, contenir des cires, des gommes, des polymères lipophiles, des produits "pâteux" ou visqueux contenant des parties solides et des parties liquides.

La phase aqueuse contient de l'eau et éventuellement un ingrédient miscible en toute proportion à l'eau comme les alcools inférieurs en C₁ à C₈ tel que l'éthanol, l'isopropanol, les polyols comme le propylène glycol, le glycérol, le sorbitol ou encore l'acétone ou l'éther.
Les émulsionnants et co-émulsionnants utilisés pour l'obtention d'une composition sous forme d'émulsion sont ceux généralement utilisés dans les domaines cosmétique et pharmaceutique. Leur nature est, en outre, fonction du sens de l'émulsion. En pratique, l'émulsionnant et éventuellement le co-émulsionnant sont présents, dans la composition, en une proportion allant de 0,1 % à 30 % en poids, de préférence de 0.2 à 20 % en poids et mieux de 0.2 à 8%. L'émulsion peut, en outre, contenir des vésicules lipidiques et notamment des liposomes.
Lorsque la composition est sous forme d'une solution ou d'un gel huileux, la phase grasse peut représenter plus de 90 % du poids total de la composition.
Avantageusement, la composition est une solution ou suspension aqueuse, alcoolique ou hydro-alcoolique et mieux une solution ou suspension eau/éthanol. La fraction alcoolique peut représenter de 5% à 99,9% et mieux de 8% à 80%.
Pour une application type mascara, la composition est une dispersion de cire-dans-eau ou de cire dans huile, une huile gélifiée, un gel aqueux, pigmenté ou non.

### Eléments additionnels de l'invention

La composition du procédé de l'invention peut comprendre, en outre, d'autres additifs cosmétiques usuellement utilisés dans les domaines concernés, choisis parmi les solvants, les épaississants ou gélifiants de phase aqueuse ou de phase huileuse, les matières colorantes solubles dans le milieu de la composition, les particules solides du type charges ou pigments, les antioxydants, les conservateurs, les parfums, les électrolytes, les neutralisants, les agents bloqueurs d'U.V. comme les filtres solaires, les polymères filmogènes, les actifs cosmétiques et pharmaceutiques à action bénéfique pour les cheveux et le cuir chevelu , des agents antipelliculaires, des actifs nutritifs, des agents anti-gras, des agents apaisants ,leurs mélanges.
Ces additifs peuvent être présents dans la composition selon les quantités généralement utilisées dans le domaine cosmétique et dermatologique et notamment à raison de 0,01 à 50% du poids total de la composition et mieux de 0,1 à 20% et par exemple de 0,1 à 10 %. Ces adjuvants, selon leur nature, peuvent être introduits dans la phase grasse, dans la phase aqueuse et/ou dans les vésicules lipidiques et notamment des liposomes.
Bien entendu l'homme du métier veillera à choisir les éventuels additifs complémentaires et/ou leur quantité de telle manière que les propriétés avantageuses de l'association selon l'invention, ne soient pas ou substantiellement pas, altérées par l'adjonction envisagée.

Comme solvants utilisables on peut citer les alcools ou,polyols inférieurs de C₂ à C₈ comme l'éthanol, l'isopropanol, le propylène glycol, le glycérol , les polyéthylèneglycols, les éthers de polyol comme le monométhyléther de dipropylènegllycol et certaines huiles cosmétiques légères comme les alcanes en C6 à C16.

Comme huiles utilisables on peut citer les huiles d'origine minérale (huile de vaseline, isoparaffine hydrogénée), les huiles d'origine végétale (fraction liquide du beurre de karité, huile de tournesol, d'abricot, de soja, d'avocat, alcool ou acide gras), les huiles d'origine animale (perhydrosqualène), les huiles de synthèse (esters d'acide gras, huile de Purcellin), les huiles siliconées (polydiméthylsiloxanes linéaires ou cycliques, phényltriméthicones) et les huiles fluorées (perfluoropolyéthers). Comme cires, on peut citer les cires siliconées, les cires d'abeille, de candellila, de riz, de carnauba, de jojoba ou de paraffine ou de polyéthylène.

Comme émulsionnants utilisables on peut citer par exemple le stéarate ou laurate de glycérol, les stéarates ou oléates de sorbitol, les alkyl diméthiconecopolyol (avec alkyle ≥ 8) et leurs mélanges pour une émulsion E/H. On peut aussi utiliser le monostéarate, isostéarate ou monolaurate de polyéthylène glycol, le stéarate ou oléate de sorbitol polyoxyéthyléné, les diméthiconecopolyols et leurs mélanges pour une émulsion H/E.
On peut aussi utiliser des tensioactifs anioniques, nonioniques, cationiques ou amphotères. Parmi les tensioactifs anioniques on mentionnera tout particulièrement les alkylsulfates, les alkyléthersulfates, les alkyléthercarboxylates.Parmi les tensioactifs non ioniques on mentionnera tout particulièrement les alkylpolyglucosides, les polysorbates , les alcools gras oxyalkylénés ou glycérolés, les triglycérides oxyalkylénés. Parmi les tensiactifs amphotères on mentionnera tout particulièrement les bétaines et les camphodiacétates. Parmi les tensioactifs cationiques on mentionnera tout particulièrement les sels d' alkyltriméthyl ammonium à chaîne grasse et les diesterquats.

Comme gélifiants hydrophiles utilisables on peut citer les polymères carboxyvinyliques (carbomer), les copolymères acryliques tels que les copolymères d'acrylates/alkylacrylates, les polyacrylamides, les polysaccharides tels que les hydroxyéthyl ou hydroxypropylcelluloses, les gommes naturelles et les argiles, et, comme gélifiants lipophiles, on peut citer les argiles modifiées comme les Bentones, les sels métalliques d'acides gras comme les stéarates d'aluminium, la silice traitée hydrophobe, l'éthylcellulose, leurs mélanges.
La composition peut contenir un actif additionnel autre que ceux cités précédemment qui peut être hydrophile et choisi parmi les protéines ou les hydrolysats de protéine, les acides aminés, l'urée, l'allantoïne, les sucres et les dérivés de sucre, les vitamines hydrosolubles, les extraits végétaux (ceux d'Iridacées ou de soja) et les hydroxy-acides (acide de fruit, acide salicylique) ; ou lipophile et choisi parmi le rétinol (vitamine A) et ses dérivés notamment ester (palmitate de rétinol), le tocophérol (vitamine E) et ses dérivés notamment ester (acétate ou palmitate de tocophérol), la vitamine F et ses esters et en particulier les esters de vitamine F et de glucose, les acides gras essentiels comme les acides linoléique, eicosatétraénoique, les acides linolénique, et eicosatriénoïque ou leurs esters et amides ; les céramides ; les huiles essentielles ;l'acide salicylique et ses dérivés comme le l'acide n-octanoyl-5 salicylique ; les esters des hydroxy acides ; les phospholipides comme la lécithine ; les antiséborrhéiques ; les antiviraux ; les antiprurigineux ; les caroténoïdes comme le β-carotène ; les lactones et leurs sels correspondants ; les phénols et polyphénols comme les flavonoïdes ; leurs mélanges.

Les agents antiandrogènes utilisables incluent notamment les inhibiteurs stéroïdiens ou non stéroïdiens de 5α-réductase, comme le finastéride et les composés décrits dans US 5 516779, l'acétate de cyprostérone, l'acide azélaïque, ses sels et ses dérivés et les composés décrits dans US 5 480913, le flutamide, les composés décrits dans les brevets US 5 411981, 5 565467 et 4 910226, ainsi que l'oxendolone, la spironolactone, le diéthylstilbestrol et la flutamide.

Les composés antimicrobiens ou antifongiques peuvent être choisis parmi les dérivés du sélénium, le kétoconazole, l'octopirox, le triclocarban, le triclosan, le pyrithione zinc, l'itraconazole, le métronidazole, le crotamiton ou les pyréthrinoïdes, l'acide asiatique, l'hinokitiol, la mipirocine, les tétracyclines, notamment l'érythromycine et les composés décrits dans EP 0680745, le chlorhydrate de clinycine, le peroxyde de benzoyle ou de benzyle et la minocycline et les composés appartenant à la classe des imidazoles tels que l'éconazole, le kétoconazole ou le miconazole ou leurs sels, les esters d'acide nicotinique, dont notamment le nicotinate de tocophérol, le nicotinate de benzyle et les nicotinates d'alkyles en C1-C6 comme les nicotinates de méthyle ou d'hexyle.

Les anti-inflammatoires peuvent être choisis parmi les anti-inflammatoires stéroïdiens comme les glucocorticoïdes, les corticostéroïdes (par exemple : l'hydrocortisone) et les anti-inflammatoires non stéroïdiens comme l'acide glycyrrhétinique et l'α-bisabolol, la benzydamine, et les composés décrits dans EP 0770399, WO 94/06434 et FR 2268523. Les rétinoïdes peuvent être choisis parmi l'acide rétinoïque, l'isotrétinoïne, l'acitrétine et le tazarotène.

Comme autres composés additionnels actifs pour favoriser la pousse et/ou limiter la chute des cheveux utilisables dans la composition selon l'invention, on peut citer le 2,4-diaminopyrimidine-3-N-oxyde (ou aminexil), le 6-0-[(9Z,12Z)-octadéca-9,12-diènoyl]hexapyranose, le chlorure de benzalkonium, le chlorure de benzéthonium, le phénol, l'oestradiol, le maléate de chlorphéniramine, les dérivés de chlorophylline, le cholestérol, la cystéine, la méthionine, le menthol, l'huile de menthe poivrée, le panthoténate de calcium, le panthénol, le résorcinol, les activateurs de la protéine kinase C, les inhibiteurs de la glycosidase, les inhibiteurs de glycosaminoglycanase, les esters d'acide pyroglutamique, les acides hexosaccharidiques ou acyl-hexosaccharique, les éthylènes aryl substitués, les amino-acides N-acylés, les flavonoïdes, les dérivés et analogues d'ascomycine, les antagonistes d'histamine, les saponines, les inhibiteurs de protéoglycanase, les agonistes et antagonistes d'estrogènes, les pseudotèrines, les cytokines et les promoteurs de facteurs de croissance, les inhibiteurs d'IL-1 ou d'IL-6, les promoteurs d'IL-10, les inhibiteurs de TNF, les benzophénones et l'hydantoïne ; les vitamines comme la vitamine D, les analogues de la vitamine B12 et le panthoténol ; les triterpènes comme l'acide ursolique et les composés décrits dans US 5529769, US 5468888, US 5631282 ; les agents antiprurigineux comme la thénaldine, la triméprazine ou la cyproheptadine ; les hormones telles que l'estriol ou ses analogues, la thyroxine et ses sels, la progestérone ; les agonistes du récepteur FP (récepteur aux prostaglandines du type F) tels que le latanoprost, le bimatoprost, le travoprost, l'unoprostone ; leurs mélanges.
On peut également envisager que la composition à application topique, soit encapsulée et notamment sous forme liposomée, telle que notamment décrite dans le document WO 94/22468. Ainsi, le composé encapsulé dans les liposomes peut être délivré sélectivement au niveau du follicule pileux.

La composition du procédé de l'invention peut être appliquée sur les zones alopéciques du cuir chevelu et des cheveux d'un individu, et éventuellement laissée en contact plusieurs heures et éventuellement rincée.
On peut, par exemple, appliquer la composition, le soir, garder celle-ci au contact toute la nuit et éventuellement effectuer un shampooing le matin. Ces applications peuvent être renouvelées quotidiennement pendant un ou plusieurs mois suivant les individus.
Avantageusement, dans le procédé selon l'invention, on applique sur les zones à traiter du cuir chevelu entre 5 et 500 µl d'une solution ou composition telle que définie précédemment.

Les exemples suivants servent à illustrer les compositions utilisées dans le procédé de l'invention sans toutefois présenter un caractère limitatif. Les quantités indiquées dans les différentes formulations sont exprimées en pourcentages en poids par rapport au poids total de la composition.

### EXEMPLES

### Exemple 1 de lotion:

| **Ingrédients** | **Quantité (% en poids)** |
|---|---|
| L-arginine | 1,5 |
| MADECASSOSIDE vendu par la société Laboratoires Roche Nicholas | 0,3 |
| PEG-40 Hydrogenated Castor Oil | 0,3 |
| Alcool éthylique 96° dénaturé | 21,9 |
| Eau désionisée | qsp 100 |

### Exemple 2 d'émulsion :

| **Ingrédients** | **Quantité (% en poids)** |
|---|---|
| L-arginine | 1,5 |
| PEG-8 Isostearate | 0,25 |
| Behenyltrimonium chloride | 0,25 |
| Huile d'avocat protégée | 0,65 |
| MADECASSOSIDE | 0,3 |
| Cire liquide de jojoba | 0,65 |
| Cyclopentadimethylsiloxane | 0,45 |
| Polydimethyl/methylaminoethyl aminopropylsiloxane en émulsion non ionique protégé | 0,75 |
| Glycérine | 0,62 |
| Alcool éthylique 96° dénaturé | 14,3 |
| Parfum | 0,4 |
| 2-amino-2-methyl-1-propanol | qs pH 7 |
| Eau désionisée | qsp100 |

## Revendications

1. Procédé de soin des fibres kératiniques humaines comprenant l'application sur ces fibres d'une composition comprenant dans un milieu physiologiquement acceptable au moins :
a) un ou plusieurs composés choisis parmi les arginines et/ou leurs sels d'acide organique ou minéral;
b) un composé ou un mélange de composés de formule (I) suivante : 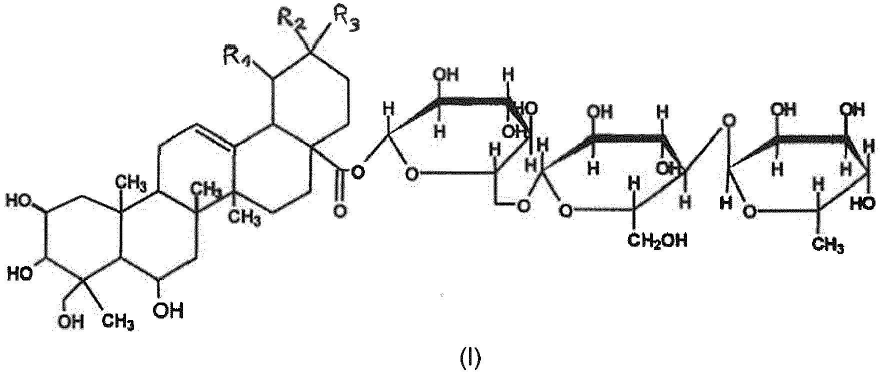 dans laquelle R₁ = H ou -CH₃ ; R₂ = H ou -CH₃ ; R₃ = -CH₃, R₁ et R₂ n'étant pas simultanément H ou bien un extrait de Centella Asiatica comprenant le composé ou le mélange de composés de formule (I).

2. Procédé selon la revendication 1, **caractérisée en ce que** le ou les composés de formule (I) est (sont) choisi(s) parmi le madécassoside et/ou le terminoloside.

3. Procédé selon la revendication 2, **caractérisée en ce que** la composition contient un mélange de madécassoside et de terminoloside.

4. Procédé selon la revendication 3, **caractérisée** en ce le mélange de madécassoside et de terminoloside a une teneur en madécassoside allant de 30 à 70% en poids par rapport au poids total du mélange et plus particulièrement 50% en poids en madécassoside.

5. Procédé selon l'une quelconque des revendications 2 à 4, **caractérisée en ce que** la composition contient un extrait de Centella Asiatica comprenant plus de 95% en poids du mélange madécassoside / terminoloside par rapport au poids de l'extrait.

6. Procédé selon l'une quelconque des revendications 1 à 5, où le composé ou le mélange de composés de formule (I) sont présents en une quantité allant de 0,001 à 20 % en poids et de préférence de 0,01 à 5 % en poids par rapport au poids total de la composition.

7. Procédé selon l'une quelconque des revendications 1 à 6, où l'arginine est choisie parmi la D-arginine, la D,L-arginine et la L-arginine.

8. Procédé selon l'une quelconque des revendications 1 à 7, où les sels d'arginine sont choisis parmi les chlorhydrates, glutamates, butyrates et glycolates.

9. Procédé selon l'une quelconque des revendications 1 à 8, où les arginines sont choisis parmi les C₁-C₄ alkyl esters de L-arginine ; les oligomères de L-arginine comprennent de 7 à 15 motifs L-arginine.

10. Procédé selon l'une quelconque des revendications 1 à 9, où l'arginine est la L-arginine.

11. Procédé selon l'une quelconque des revendications 1 à 10, où la ou les arginines et/ou leurs sels sont présents dans une quantité allant de 0,01% à 5% du poids total de la composition et plus préférentiellement en une quantité représentant de 0,1% à 5% du poids total de la composition, et encore plus préférentiellement de 1 % à 5 %.

12. Procédé selon l'une quelconque des revendications 1 à 11, comprenant l'application sur ces fibres d'une composition comprenant en outre, au moins un additif cosmétique choisi parmi les solvants, les épaississants ou gélifiants de phase aqueuse ou de phase huileuse, les matières colorantes solubles dans le milieu de la composition, les particules solides du type charges ou pigments, les antioxydants, les conservateurs, les parfums, les électrolytes, les neutralisants, les agents bloqueurs d'U.V, les polymères filmogènes, les actifs cosmétiques et pharmaceutiques à action bénéfique pour les cheveux et le cuir chevelu , des agents antipelliculaires, des actifs nutritifs, des agents anti-gras, des agents apaisants, leurs mélanges.

13. Procédé selon l'une quelconque des revendications 1 à 12, comprenant l'application sur ces fibres d'une composition comprenant en outre, au moins un composé additionnel favorisant la stimulation et/ou l'induction de la croissance des cheveux et/ou limitant la chute des fibres kératiniques humaines.

14. Utilisation cosmétique de l'association d'un ou plusieurs composés choisis parmi les arginines et/ou leurs sels d'acide organique ou minéral; et d'un composé ou d'un mélange de composés de formule (I) ou bien d'un extrait de Centella Asiatica comprenant le composé ou le mélange de composés de formule (I) tels que définis dans les revendications précédentes dans une composition de soin des fibres kétatiniques humaines, dans le but d'induire et/ou stimuler la croissance des fibres kératiniques humaine et/ou freiner leur chute et/ou augmenter leur densité.

15. Procédé de traitement cosmétique des fibres kératiniques humaines et/ou du cuir chevelu, destiné à stimuler et/ou induire la croissance desdites fibres en particulier les cheveux et les cils et/ou freiner leur chute et/ou augmenter leur densité, **caractérisé par le fait qu'**il consiste à appliquer sur ces fibres et/ou le cuir chevelu, une composition cosmétique telle que définie dans le procédé de l'une quelconque des revendications 1 à 13, à laisser celle-ci au contact de ces fibres kératiniques et/ou du cuir chevelu, et éventuellement à rincer ces fibres kératiniques et/ou le cuir chevelu.

## Patentansprüche

1. Verfahren zur Pflege menschlicher Keratinfasern, im Rahmen dessen auf diese Fasern eine Zusammensetzung aufgebracht wird, die in einem physiologisch unbedenklichen Milieu mindestens Folgendes umfasst:
a) eine oder mehrere Verbindungen, die aus den Argininen und/oder Salzen ausgewählt sind, welche diese mit organischen Säuren oder Mineralsäuren bilden;
b) eine Verbindung oder eine Mischung von Verbindungen der folgenden Formel (I) 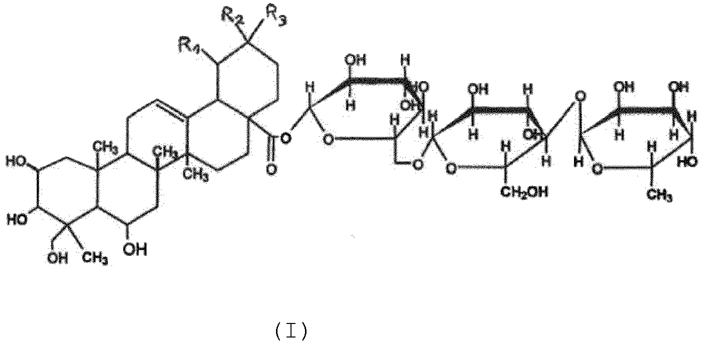 in welcher R₁ = H oder -CH₃ ist; R₂ = H oder -CH₃ ist; R₃ = -CH₃ ist, wobei R₁ und R₂ nicht gleichzeitig gleich H sind, oder aber einen Extrakt von Centella Asiatica, der die Verbindung oder die Mischung von Verbindungen der Formel (I) umfasst.

2. Verfahren nach Anspruch 1, **dadurch gekennzeichnet, dass** die Verbindung (en) der Formel (I) aus Madecassosid und/oder Terminolosid ausgewählt ist/sind.

3. Verfahren nach Anspruch 2, **dadurch gekennzeichnet, dass** die Zusammensetzung eine Mischung aus Madecassosid und aus Terminolosid enthält.

4. Verfahren nach Anspruch 3, **dadurch gekennzeichnet, dass** diese Mischung aus Madecassosid und aus Terminolosid einen Gehalt an Madecassosid im Bereich von 30 bis 70 Gewichts-%, bezogen auf das Gesamtgewicht der Mischung, und insbesondere von 50 Gewichts-% an Madecassosid aufweist.

5. Verfahren nach einem beliebigen der Ansprüche 2 bis 4, **dadurch gekennzeichnet, dass** die Zusammensetzung einen Extrakt von Centella Asiatica enthält, der mehr als 95 Gewichts-% der Madecassosid/Terminolosid-Mischung umfasst, bezogen auf das Gewicht des Extrakts.

6. Verfahren nach einem beliebigen der Ansprüche 1 bis 5, wobei die Verbindung oder die Mischung von Verbindungen der Formel (I) in einer Menge im Bereich von 0,001 bis 20 Gewichts-%, und vorzugsweise von 0,01 bis 5 Gewichts-%, bezogen auf das Gesamtgewicht der Zusammensetzung vorliegen.

7. Verfahren nach einem beliebigen der Ansprüche 1 bis 6, wobei das Arginin aus D-Arginin, D,L-Arginin und L-Arginin ausgewählt ist.

8. Verfahren nach einem beliebigen der Ansprüche 1 bis 7, wobei die Argininsalze aus den Chlorhydraten, Glutamaten, Butyraten und Glykolaten ausgewählt sind.

9. Verfahren nach einem beliebigen der Ansprüche 1 bis 8, wobei die Arginine aus den C₁-C₄-Alkylestern des L-Arginins; den Oligomeren von L-Arginin, welche 7 bis 15 L-Arginin-Bausteine umfassen, ausgewählt sind.

10. Verfahren nach einem beliebigen der Ansprüche 1 bis 9, wobei es sich bei dem Arginin um L-Arginin handelt.

11. Verfahren nach einem beliebigen der Ansprüche 1 bis 10, wobei das oder die Arginine und/oder deren Salze in einer Menge im Bereich von 0,01 % bis 5 % des Gesamtgewichts der Zusammensetzung vorliegen, und stärker bevorzugt in einer Menge, die 0,1 % bis 5 % des Gesamtgewichts der Zusammensetzung und noch stärker bevorzugt 1 % bis 5 % ausmacht.

12. Verfahren nach einem beliebigen der Ansprüche 1 bis 11, im Rahmen dessen auf die Fasern eine Zusammensetzung aufgebracht wird, die weiterhin mindestens einen kosmetischen Zusatzstoff umfasst, welcher aus den Lösungsmitteln, den Verdickungsmitteln oder Gelbildnern für Wasserphasen oder Ölphasen, den Farbstoffen, die im Milieu der Zusammensetzung löslich sind, den feststofflichen Partikeln des Typs Füllstoffe oder Pigmente, den Antioxidantien, den Konservierungsstoffen, den Duftstoffen, den Elektrolyten, den Neutralisierungsmitteln, den UVblockierenden Mitteln, den filmbildenden Polymeren, den kosmetischen und pharmazeutischen Wirkstoffen, die eine günstige Wirkung auf das Kopfhaar und die Kopfhaut ausüben, den Antischuppenmitteln, den nährenden Wirkstoffen, den fettbekämpfenden Wirkstoffen, den beruhigenden Wirkstoffen und deren Mischungen ausgewählt ist.

13. Verfahren nach einem beliebigen der Ansprüche 1 bis 12, im Rahmen dessen auf die Fasern eine Zusammensetzung aufgebracht wird, die weiterhin mindestens eine zusätzliche Verbindung enthält, welche die Anregung und/oder Einleitung des Kopfhaarwachstums begünstigt und/oder das Ausfallen menschlicher Keratinfasern begrenzt.

14. Kosmetische Verwendung der Kombination aus einer oder mehreren Verbindungen, die aus den Argininen und/oder Salzen ausgewählt sind, welche diese mit organischen Säuren oder Mineralsäuren bilden; und aus einer Verbindung oder einer Mischung von Verbindungen der Formel (I) oder aber einem Extrakt von Centella Asiatica, der die Verbindung oder die Mischung von Verbindungen der Formel (I) gemäß den Begriffsbestimmungen in den vorhergehenden Ansprüchen umfasst, in einer Zusammensetzung zur Pflege menschlicher Keratinfasern, um das Wachstum der menschlichen Keratinfasern einzuleiten und/oder anzuregen und/oder um deren Ausfall zu verzögern und/oder um deren Dichte zu erhöhen.

15. Verfahren zur kosmetischen Behandlung menschlicher Keratinfasern und/oder der Kopfhaut, welches dazu bestimmt ist, das Wachstum der Fasern, insbesondere des Kopfhaars und der Wimpern, anzuregen und/oder einzuleiten und/oder deren Ausfall zu bremsen und/oder deren Dichte zu erhöhen, **dadurch gekennzeichnet, dass** es darin besteht, eine kosmetische Zusammensetzung gemäß der Begriffsbestimmung im Verfahren eines beliebigen der Ansprüche 1 bis 13 auf die Fasern und/oder die Kopfhaut aufzubringen, sie mit diesen Keratinfasern und/oder mit der Kopfhaut in Kontakt zu belassen und möglicherweise die Keratinfasern und/oder die Kopfhaut zu spülen.

## Claims

1. Care process for human keratin fibres, comprising the application, to said fibres, of a composition comprising, in a physiologically acceptable medium, at least:
a) one or more compounds chosen from arginines and/or the organic or inorganic acid salts thereof;
b) a compound or a mixture of compounds of formula (I) below: 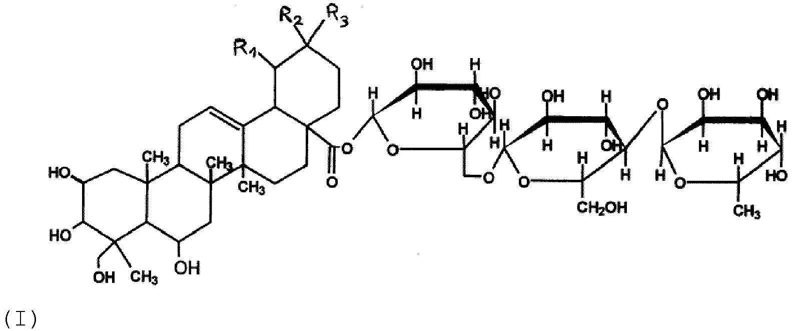 in which R₁ = H or -CH₃; R₂ = H or -CH₃; R₃ = -CH₃, R₁ and R₂ not being simultaneously H, or else an extract of *Centella asiatica* comprising the compound or the mixture of compounds of formula (I).

2. Process according to Claim 1, **characterized in that** the compound(s) of formula (I) is (are) chosen from madecassoside and/or terminoloside.

3. Process according to Claim 2, **characterized in that** the composition contains a mixture of madecassoside and of terminoloside.

4. Process according to Claim 3, **characterized in that** the mixture of madecassoside and of terminoloside has a madecassoside content ranging from 30% to 70% by weight relative to the total weight of the mixture, and more particularly 50% by weight of madecassoside.

5. Process according to any one of Claims 2 to 4, **characterized in that** the composition contains an extract of *Centella asiatica* comprising more than 95% by weight of the madecassoside/terminoloside mixture, relative to the weight of the extract.

6. Process according to any one of Claims 1 to 5, wherein the compound or the mixture of compounds of formula (I) is present in an amount ranging from 0.001% to 20% by weight, and preferably from 0.01% to 5% by weight, relative to the total weight of the composition.

7. Process according to any one of Claims 1 to 6, wherein the arginine is chosen from D-arginine, D,L-arginine and L-arginine.

8. Process according to any one of Claims 1 to 7, wherein the arginine salts are chosen from hydrochlorides, glutamates, butyrates and glycolates.

9. Process according to any one of Claims 1 to 8, wherein the arginines are chosen from the C₁-C₄ alkyl esters of L-arginine; and the oligomers of L-arginine comprise from 7 to 15 L-arginine units.

10. Process according to any one of Claims 1 to 9, wherein the arginine is L-arginine.

11. Process according to any one of Claims 1 to 10, wherein the arginine(s) and/or the salts thereof are present in an amount ranging from 0.01% to 5% of the total weight of the composition and more preferably in an amount representing from 0.1% to 5% of the total weight of the composition, and even more preferably from 1% to 5%.

12. Process according to any one of Claims 1 to 11, comprising the application, to said fibres, of a composition also comprising at least one cosmetic additive chosen from solvents, aqueous-phase or oily-phase thickeners or gelling agents, dyestuffs that are soluble in the medium of the composition, solid particles of the filler or pigment type, antioxidants, preservatives, fragrances, electrolytes, neutralizing agents, UV blockers, film-forming polymers, cosmetic and pharmaceutical active agents with a beneficial action on the hair and the scalp, antidandruff agents, nutritive active agents, agents for combating grease, and calmatives, and mixtures thereof.

13. Process according to any one of Claims 1 to 12, comprising the application, to said fibres, of a composition also comprising at least one additional compound for promoting the stimulation and/or induction of hair growth and/or limiting the loss of human keratin fibres.

14. Cosmetic use of the combination of one or more compounds chosen from arginines and/or the organic or inorganic acid salts thereof; and of a compound or of a mixture of compounds of formula (I) or else of an extract of *Centella asiatica* comprising the compound or the mixture of compounds of formula (I) as defined in the preceding claims, in a care composition for human keratin fibres, with the aim of inducing and/or stimulating the growth of human keratin fibres and/or preventing their loss and/or increasing their density.

15. Cosmetic treatment process for human keratin fibres and/or the scalp, for stimulating and/or inducing the growth of said fibres, in particular the hair and the eyelashes, and/or preventing their loss and/or increasing their density, **characterized in that** it comprises applying, to these fibres and/or the scalp, a cosmetic composition as defined in the process of any one of Claims 1 to 13, leaving said composition in contact with these keratin fibres and/or with the scalp, and optionally rinsing these keratin fibres and/or the scalp.
